# DEMANDE DE BREVET EUROPEEN

(11) **EP 0 819 698 A2**
(43) Date de publication de la demande: **21.01.1998**
(21) Numéro de dépôt: 97401197.5
(22) Date de dépôt: 30.05.1997
(51) Int. Cl.: C07H 15/04, C11D 1/66, A61K 7/075, A61K 7/18, A61K 31/70, B01F 17/00, C09K 19/34

(54) **Acides fluoroalkyl glycosides uroniques et lactones-6(3) correspondantes, préparation et utilisations**

(30) Priorité: 20.06.1996 FR 9607693
(71) Demandeur: CECA S.A., 92800 Puteaux (FR)
(72) Inventeur: Petit, Serge, 74540 Cusy (FR); Fouquay, Stéphane, 76130 Mont-Saint-Aignan (FR); Bernard, Daniel, 92400 Courbevoie (FR)
(74) Mandataire: Haicour, Philippe

(57) **Abrégé**

L'invention a pour objet des dérivés fluoroalkylés d'acide uroniques de formule : 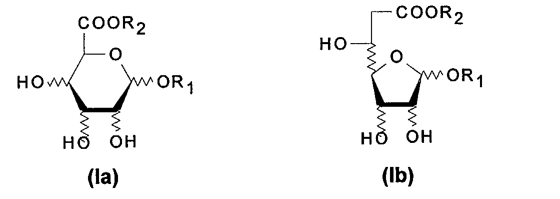 et les lactones correspondantes.

Elle concerne également un procédé de préparation desdits composés qui consiste à faire réagir un acide glycosiduronique ou un glucosiduronolactone avec un alcool de formule R₁OH, ce procédé peut comporter en outre des étapes de basification et d'acidification.

Les composés selon l'invention peuvent être utilisés notamment comme agent tensio-actif.

## Description

La présente invention a pour objet des acides fluoroalkylglycosides uroniques et les lactones correspondantes, leur procédé de préparation et leurs utilisations, notamment en tant qu'agent tensio-actif.

Les composés fluorés sont connus pour leurs propriétés tensio-actives. La famille la plus importante de ces composés est représentée par les dérivés anioniques du type carboxylate, sulfonate, sulfate et phosphate.

En particulier, les composés fluorés à base de sucre ont fait l'objet de nombreux travaux car ils présentent, entre autres, une excellente compatibilité avec les tissus humains. De ce fait, leurs applications relèvent essentiellement du domaine biomédical où ils sont employés pour former des émulsions ou des vésicules fluorocarbonées agissant comme substitut sanguin, agent de contraste ou agent de vectorisation et de relargage contrôlés de médicaments.

L'art antérieur dans ce domaine comprend notamment :
- l'article de RIESS J.G. et GREINIER J. [Carbohydrates as Organic Raw Materials II, pp. 209-259, Ed. VCH (1993)] qui décrit des sucres perfluoroalkylés comprenant une tête hydrophile de nature glucidique, un élément de jonction (par exemple ester, éther, amide ou phosphoester), un espaceur hydrocarboné et une queue perfluoroalkylée;
- les demandes de brevet WO-A-92 05444 et WO-A-05445 qui décrivent un réactif destiné à immobiliser une biomolécule sur un support fluorocarboné solide ou liquide. Ce réactif comporte, d'une part, des groupements fluoroalkyles permettant la fixation sur le support et, d'autre part, un groupement réactif sur le carbone anomérique nécessaire au couplage covalent avec la biomolécule ;
- la demande WO-A-92 21688 qui décrit des molécules amphiphiles perfluoroalkylées et phosphorées comportant notamment un radical dérivé d'un sucre ;
- et la demande WO-A-94 03468 qui divulgue des molécules amphiphiles comportant une partie hydrophile polyhydroxylée, notamment un mono- ou oligosaccharide, une partie hydrophobe, par exemple fluorocarbonée, et un élément de jonction desdites parties dérivé d'un acide aminé ou d'un peptide.

Par ailleurs, on sait que les dérivés d'acide glycosiduroniques sont des tensio-actifs performants et bien tolérés par la peau et les muqueuses. De tels composés sont par exemple décrits dans EP-A-550276, EP-A-537 820, EP-A-532 370 et JP-A-05170642. Il n'est cependant pas enseigné que les composés précités puissent contenir un ou plusieurs radicaux fluoroalkyles.

Il a maintenant été trouvé que des dérivés fluorés d'acides glycosides uroniques présentent des propriétés tensio-actives. La présente invention concerne donc des acides perfluoroalkylglycosides uroniques et leurs dérivés, ainsi que les lactones correspondantes.

Un autre objet de l'invention concerne un procédé de préparation des acides et lactones précités par greffage d'une chaîne fluoroalkyle (glycosylation et, le cas échéant estérification). Contrairement aux procédés de l'art antérieur cités ci-avant qui font intervenir des étapes de protection et de déprotection des groupements hydroxyles, le présent procédé fait intervenir un substrat non protégé.

Un autre objet de l'invention concerne enfin l'utilisation desdits acides et lactones en tant qu'agent tensio-actif.

Plus précisément, les acides perfluoroalkylglycosides uroniques et leurs dérivés de la présente invention, on pour formule : 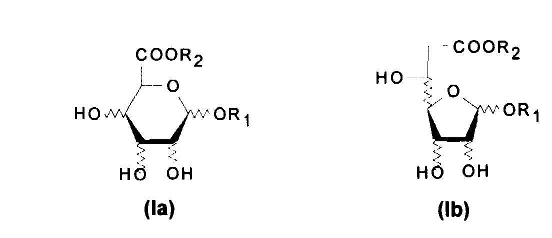 dans laquelle :
R₁ représente un radical fluoroalkyle, linéaire ou ramifié, saturé ou insaturé renfermant 4 à 46 atomes de carbone ;
R₂ représente H, R₁ tel que défini ci-avant, un métal alcalin ou alcalino-terreux, ou un ammonium quaternaire de formule : 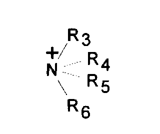 dans laquelle R₃, R₄, R₅ et R₆, identiques ou différents représentent H, un radical alkyle ou hydroxyalkyle en C₁C₆.

De préférence, R₁ a pour formule :

-(CH₂)ₘ-(CH=CH)ₙ-(CF₂)ₚ-CF₃

dans laquelle :
m est compris entre 2 et 22,
n est égal à 0 ou 1,
p est compris entre 1 et 21.

Les lactones correspondant aux acides fluoroalkylglycosides uroniques, également objet de la présente invention, ont pour formule : 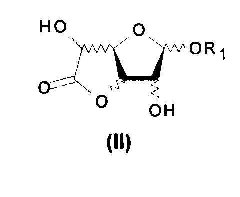 dans laquelle R₁ a la signification donnée ci-avant.

Les lactones préférées sont la fluoroalkyl glucofuranuronolactone-6(3) et la fluoroalkyl mannofuranuronolactone-6(3), la fluoroalkyl idofuranuronolactone-6(3), la fluoroalkyl gulofuranuronolactone-6(3), et mieux encore la fluoroalkyl D-glucofuranuronolactone-6(3), la fluoroalkyl D-mannofuranuronolactone-6(3), la fluoroalkyl L-idofuranuronolactone-6(3) et la fluoroalkyl L-gulofuranuronolactone-6(3).

Les acides fluoroalkylglycosides uroniques et leurs dérivés ainsi que les lactones correspondantes peuvent être préparés selon le procédé qui consiste à faire réagir un acide glycosiduronique ou une glycosiduronolactone avec un alcool de formule R₁OH dans laquelle R₁ a la signification donnée ci-avant, en présence d'un catalyseur acide, récupérer l'acide fluoroalkyl glycosiduronate, ses dérivés ou la lactone correspondante et, le cas échéant mettre en oeuvre une base afin d'obtenir le sel de l'acide fluoroalkylglycosiduronique.

Le procédé selon l'invention sera mieux compris à la lumière des explications qui suivent.

### • Préparation de fluoroalkylglycosiduronates de fluoroalkyle (R₂ = fluoroalkyle C₄-C₄₆) et de fluoroalkylgycosiduronolactone

Le procédé consiste à faire réagir :
- 1 équivalent en mole d'un acide glycosiduronique ou d'une glycosiduronolactone ;
- 1 à 25 équivalents molaires d'un alcool de formule R₁OH, R₁ ayant la signification donnée précédemment, de préférence 1 à 3 équivalents dans le cas où on utilise un solvant additionnel et 3 à 25 équivalents lorsqu'aucun solvant additionnel n'est employé ;
- 0,01 à 2 équivalents molaires d'un catalyseur acide ou 0,05 à 6 équivalents en poids d'une résine ou d'une argile acide.

L'acide glycosiduronique est généralement choisi parmi les acides uroniques renfermant 5 ou 6 atomes de carbone tels que les acides riburonique, arabinuronique, xyluronique, lyxuronique, glucuronique, galacturonique, mannuronique, iduronique, guluronique, alluronique, talluronique et altruronique. De préférence, on utilise l'acide glucuronique, galacturonique, mannuronique, iduronique ou guluronique, et mieux encore, l'acide glucuronique ou galacturonique.

La glycosiduronolactone est généralement choisie parmi les lactones des acides glycosiduroniques précités. A titre d'exemple, on peut citer la glucuronolactone-6(3), la mannuronolactone-6(3), la guluronolactone-6(3) et l'iduronolactone-6(3).

De préférence, on utilise la glucuronolactone-6(3) et la mannuronolactone-6(3).

Le solvant additionnel pouvant être utilisé dans le procédé selon l'invention est généralement choisi parmi les étheroxydes tels que le tétrahydrofuranne, l'éther diméthylique de l'éthylène glycol, les hydrocarbures halogénés tels que le dichlorométhane, le dichloroéthane, le chloroforme, les esters tels que l'acétate d'éthyle, de propyle ou de butyle, les alcools tels que le méthanol, l'éthanol, le propanol ou le butanol, les amides tels que le N-méthylformamide ou le N,N-diméthylformamide et les mélanges de ces composés.

Le solvant additionnel est généralement mis en oeuvre à raison de 0 à 20 équivalents en poids pour un équivalent en poids d'acide glycosiduronique ou de glycosiduronolactone de départ.

Le catalyseur est généralement choisi parmi les acides tels que l'acide chlorhydrique ou sulfurique, les acides alkylsulfuriques tels que l'acide décyl ou laurylsulfurique, les acides sulfoniques tels que l'acide benzène-sulfonique, paratoluène-sulfonique ou camphro-sulfonique, les acides alkyl-sulfoniques tels que l'acide méthane-sulfonique, l'acide décyl- ou lauryl-sulfonique, les acides sulfosucciniques, les sulfosuccinates d'alkyle tels que le sulfosuccinate de décyle ou de lauryle, les acides perhalohydriques tels que l'acide perchlorique ou l'acide hypophosphoreux, et les mélanges de ces acides. De préférence, on utilise l'acide sulfurique, un acide alkylsulfurique, l'acide méthane-sulfonique, l'acide sulfosuccinique, un sulfosuccinate d'alkyle et l'acide hypophosphoreux.

Le catalyseur peut également être choisi parmi les métaux tels que le cuivre ou le fer, les oxydes métalliques, les sels métalliques tels que les halogénures, les halogènes tels que l'iode, les pentahalogénures d'antimoine tels que les pentachlorures ou les pentafluorures, et les sulfates de titane.

La résine est généralement choisie parmi les résines se présentant sous la forme H⁺, par exemple les résines sulfoniques.

La résine et l'argile acides présentent, en outre, une forte capacité de rétention de l'eau. Elles sont pour cela préférées lorsqu'un effet déshydratant est recherché. On peut les utiliser seules ou en mélange avec un agent de déshydratation classique, par exemple un tamis moléculaire ou une zéolite.

Le procédé est généralement mis en oeuvre à une température comprise entre 50 et 200°C, de préférence 70 et 150°C, et pendant une durée pouvant varier de une heure à trois jours, de préférence 1 à 24 heures.

La réaction est généralement effectuée sous une pression comprise entre 0,013 et 101,325 KPa et de préférence entre 0,013 et 4 KPa.

A l'issue de la réaction, on effectue une étape d'élimination du catalyseur qui peut être réalisée de deux manières selon la nature de ce dernier.

Lorsque le catalyseur est un acide (catalyse en milieu homogène), on le neutralise, par exemple au moyen d'hydrogénocarbonate d'un métal alcalin tel que le sodium ou le potassium, et on élimine le sel formé par filtration ou par précipitation dans de l'eau et lavage du précipité avec de l'eau, le cas échéant un solvant choisi parmi les solvants additionnels précités, de préférence un alcane ou un étheroxyde.

Lorsque le catalyseur est une résine ou une argile (catalyse hétérogène), il est éliminé par filtration conventionnelle.

Après ladite étape d'élimination du catalyseur et, le cas échéant, de l'excès d'alcool, on récupère le fluoroalkylglycosiduronate de fluoroalkyle ou la fluoroalkylglycosiduronolactone.

Ces composés sont le plus souvent constitués d'un mélange des formes pyranique et furanique qui peuvent être séparées, par exemple par chromatographie sur une colonne de silice.

### • Préparation des sels d'acides fluoroalkylglycosides uroniques (R₂ = métal alcalin ou alcalino-terreux, ammonium quaternaire)

Le procédé de préparation de ces composés consiste à faire réagir:
- un équivalent molaire de fluoroalkylglycosiduronate de fluoroalkyle ou de fluoroalkylglycosidurolactone obtenu selon le mode de préparation décrit ci-avant ;
- 0,5 à 10 équivalents molaires, de préférence 1 à 3 équivalents d'une base.

La base utilisée est généralement choisie parmi les carbonates, les hydrogénocarbonates, par exemple d'un métal alcalin tel que le sodium ou le potassium, les composés de formule M(OH)ₓ dans laquelle M représente un métal alcalin ou alcalino-terreux et x est la valence du métal, les alumines basiques et les hydroxydes d'ammonium quaternaires de formule : 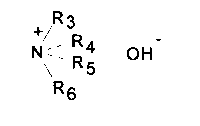 dans laquelle R₃, R₄, R₅ et R₆, identiques ou différents ont la signification donnée précédemment. De préférence, on utilise l'hydroxyde de sodium ou de potassium, l'ammoniaque ou un hydroxyde d'alkyl (hydroxyalkyl)ammonium.

Le procédé peut également mettre en oeuvre d'autres constituants, tels que :
- un solvant (0 à 20 équivalents en poids pour un équivalent en poids de glycoside engagé) choisi parmi l'eau, les alcanes tels que le pentane, l'hexane, l'heptane et l'octane, les étheroxydes tels que le tétrahydrofuranne, le dioxanne et l'éther diméthylique de l'éthylène ou du diéthylène glycol, les hydrocarbures halogénés tels que le dichlorométhane, le chloroforme et le dichloroéthane, les alcools à chaîne courte tels que le méthanol, l'éthanol, le propanol, l'isopropanol et le butanol, ou à chaîne plus longue jusqu'à 14 atomes de carbone) tels que l'octanol, le décanol, le dodécanol et le tétradécanol, et les mélanges de ces solvants.

De préférence, on utilise l'eau, le pentane, l'hexane, l'heptane, le tétrahydrofuranne, le méthanol, l'éthanol et l'isopropanol.
- un catalyseur de transfert de phase (0 à 1 % en poids pour 1 équivalent de glycoside engagé), par exemple un agent tensio-actif non ionique, cationique ou anionique, notamment les amines et les composés contenant des ions d'ammonium ou phosphonium, un éther couronne, un cryptand, un amino polyéther, un phosphorylsulfoxide et un ester de glycol ou de sorbitan.

A titre d'exemple de composés de type ammonium, on peut citer le chlorure d'hexadécyltriméthylammonium, le bromure ou le chlorure de tétrabutylammonium, le chlorure de benzyltriméthylammonium, le chlorure de trioctylammonium, le chlorure de tricapryl méthylammonium, et le sulfate acide de tétrabutylammonium.

De préférence, on utilise le chlorure d'hexadécyltriméthylammonium et le bromure ou le chlorure de tétrabutylammonium.

Le procédé est généralement mis en oeuvre à une température comprise entre 0 et 100°C, de préférence 0 et 60°C, et pendant une durée pouvant varier de 15 à 360 minutes, de préférence entre 15 et 120 minutes.

A l'issue de la réaction, éventuellement après élimination de l'alcool formé et du solvant, on récupère le sel d'acide fluoroalkylglycoside uronique.

Ce sel peut, le cas échéant, être lavé avec un solvant choisi parmi les solvants de réaction précités, et séché. ll peut en outre, subir un traitement destiné à éliminer une éventuelle coloration indésirable, par exemple au moyen d'un charbon actif ou d'une résine absorbante.

### • Préparation des acides fluoroalkylglycosides uroniques (R₂ = H)

Le procédé consiste à acidifier le sel d'acide fluoroalkylglycoside uronique obtenu selon le mode de préparation décrit ci-avant par un acide.

A titre d'exemple d'un tel acide, on peut citer l'acide chlorhydrique, l'acide sulfurique, un acide sulfonique ou une résine sulfonique sous forme H⁺.

On utilise généralement 1 à 1,2 équivalent molaire pour un équivalent molaire de glycoside de départ.

Les composés selon l'invention présentent des propriétés dispersantes, mouillantes, moussantes, émulsionnantes, nettoyantes et de solubilisation permettant leur emploi en tant qu'agent tensio-actif.

Ces composés peuvent notamment être employés comme détergent ou adjuvant de détergence par exemple dans des compositions de lavage des sols et des vitres, et pour le nettoyage des composants électroniques.

Ils peuvent également entrer dans des compositions de traitements capillaires et des shampooings et après-shampooings. Du fait de leurs propriétés émulsifiantes, lubrifiantes et/ou oléophobes, ils peuvent être employés en cosmétologie pour préparer des pommades, des crèmes et des laits de beauté. En outre, leur pouvoir moussant et adoucissant présente un intérêt pour la fabrication de bains moussants.

Les composés selon l'invention peuvent intervenir dans la préparation de produits pour l'hygiène buccale tels que les dentifrices et les lotions de pré- et post-brossage des dents.

Les propriétés de mouillage peuvent être mises à profit dans le traitement anti-buée ou antistatique, et le polissage des matériaux divers, par exemple, le verre, le métal et le plastique.

Les composés selon l'invention présentent un intérêt dans la préparation de compositions de poudres et de mousses anti-feu.

Ils peuvent aussi être incorporés dans des adhésifs à base d'eau ou de solvants organiques, des revêtements de surface tels que les peintures et les vernis, des ciments, et des cires ou des graisses de lubrification.

Les composés de l'invention peuvent être utilisés dans des domaines variés où ils peuvent agir en tant que :
- additif de bains chromiques, d'imperméabilisation notamment pour le papier, d'émulsions photographiques, des polymères notamment pour préparer des films plastiques ;
- substitut du mercure dans les batteries à anode de zinc ;
- agent de polymérisation en dispersion ou en émulsion, notamment de composés carbonés chlorés ou fluorés, et intervenir dans les procédés électrochimiques;
- cristaux liquides pouvant être utilisés en électrooptique ou en pharmacie pour la formation de capsules, de vésicules ou de phases lamellaires permettant de stabiliser et de vectoriser des principes actifs, et
- transporteur d'oxygène dans des compositions à usage biomédical.

Dans les exemples qui suivent, on utilise les méthodes d'analyse ci-après :
- Résonance magnétique nucléaire (RMN)

Les déplacements chimiques sont exprimés en ppm et les constantes de couplage J en Hz.
- RMN¹H : réalisée à 300 MHz en présence d'acétone-d₆ contenant 5 % en poids de D₂O (exemples 1, 4 et 7-anomère β) ou de CDCl₃ (exemple 9), ou à 200 MHz en présence d'acétone-d₆ contenant 5 % en poids de D₂O (exemple 7-anomère α).
- RMN¹³C : réalisée à 300 MHz en présence d'acétone-d₆ (exemples 1, 4 et 7) ou à 250 MHz en présence de CDCl₃ (exemple 9).

- Spectrographie infrarouge (IR) ; pastille KBr 1 %

Les valeurs sont exprimées en cm⁻¹.
- Le Rf est mesuré par chromatographie sur couche mince de silice (épaisseur du film : 200 µm ; taille des particules : 5-10 µm). Le solvant de migration est un mélange chloroforme/méthanol 9/5 (v/v) (exemple 9) ou l'acétate d'éthyle (exemple 12). Les spots de migration sont révélés par pulvérisation d'acide sulfurique à 50 % en volume dans l'eau et chauffage à 120°C pendant 2 minutes.
- Tension superficielle (γ_{CMC}) : mesurée à 25°C à l'aide d'un tensiomètre LAUDA selon la méthode d'arrachement à l'étrier (norme ISO.304) à partir d'une solution aqueuse dans de l'eau bidistillée. Les résultats sont exprimés en mN/m. Le composé de référence utilisé est une polyfluoroalkyl bétaïne (FORAFAC 1157 commercialisé par ELF ATOCHEM S.A.).
- Concentration micellaire critique (CMC): mesurée selon la norme ISO.4311. Les résultats sont exprimés en g/l.

### EXEMPLE 1

### Préparation de 2-(perfluorohexyl)éthyl D-glucofuranuronolactone-6(3).

Dans un ballon tricol de 500 ml, on introduit 8,8 g (50 mmoles) de D-glucuronolactone-6(3) (85, 145-0 ; ALDRICH), 109,2 g (300 mmoles) de 2-(perfluorohexyl)éthanol (FORALKYL EOH 6; CECA S.A.) et 0,24 g (2,5 mmoles) d'acide méthanesulfonique.

Après 6 heures à 95°C sous un vide de 13,33 KPa, le milieu réactionnel devenu homogène est refroidi à 20°C, neutralisé avec du NaHCO₃ et filtré. Le filtrat est récupéré et concentré à 90°C sous vide (0,133 KPa). Le résidu visqueux ainsi obtenu (30 g) est solubilisé dans 6 ml d'acétate d'éthyle pour obtenir une huile fluide (35,5 g) qui est chromatographiée sur une colonne (silice 230-400 Mesh ASTM ; éluant: acétate d'éthyle/hexane 65/35 (v/v). On récupère 9,2 g de 2-(perfluorohexyl)éthyl β-D-glucofuranuronolactone-6(3) (Rf= 0,3) et 1 g de 2-(perfluorohexyl)éthyl α-D-glucofuranuronolactone-6(3) (Rf = 0,5), soit un rendement calculé sur la base de la D-glucuronolactone-6(3) de départ égal à 35 % et 3,8 % respectivement.

Les caractéristiques des lactones obtenues sont présentées ci-après :

| RMN¹H | | |
|---|---|---|
| | **Anomère α** | **Anomère β** |
| H1 | 5,30 ; d | 5,10 ; s |
| | J_{H1H2} = 4,56 | J_{H1H2} < 1,5 |
| | | |
| H4 | 4,84 ; m | 4,98 ; dxd |
| | | J_{H4H5} = 6,37 |
| | | J_{H4H3} = 4,74 |
| | | |
| H3 | 4,77 ; m | 4,84 ; d |
| | | J_{H3H4} = 4,74 |
| | | J_{H3H2} < 1 |
| | | |
| H5 | 4,62 ; d | 4,55 ; d |
| | J_{H5H4} = 5,23 | J_{H5H4} = 6,37 |
| | | |
| H2 | 4,27 ; dxd | 4,23 ; s |
| | J_{H1H2} = 4,56 | J_{H2H1} < 1,5 |
| | J_{H2H3} < 1 | J_{H2H3} < 1 |
| -CH₂-O- | 4,20-4,10 ; m et 4,00-3,90 ; m | 4,20-4,10 ; m et 3,74-3,64 ; m |
| | | |
| -CH₂-CH₂-O- | 2,80-2,55 | 2,70-2,20 ; m |

| RMN¹³C | | |
|---|---|---|
| | **Anomère α** | **Anomère β** |
| C = O | 175,25 | 175,54 |
| C1 | 104,13 | 109,68 |
| C2 | 85,41 | 83,97 |
| C3 | 77,39 | 79,29 |
| C4 | 77,87 | 78,87 |
| C5 | 70,58 | 70,11 |
| -CH₂-O- | 61,45 et 61,54 | 60,25 et 60,12 |
| -CF₂-CH₂- | 32,23-31,39 | 32,13-31,71 |

| IR | | |
|---|---|---|
| | **Anomère α** | **Anomère β** |
| δCH₂ | 2963 | 2933 |
| γC = O | 1768 | 1805, 1777 et 1756 |
| γ-OH | 3398 | 3403 |

### EXEMPLE 2

### Préparation de 2-(perfluorohexyl)éthyl β-D-glucofuranuronate de sodium.

A 1 g (1,9mmole) de 2-(perfluorohexyl)éthyl **β**-D-glucofuranosiduronolactone-6(3) de l'exemple 1 solubilisée dans 10 ml d'éthanol absolu, on ajoute par fraction de la lessive de soude jusqu'à obtention d'un pH égal à 12.

Après 2 heures d'agitation à une température de 20°C, le précipité formé est filtré, lavé à l'éthanol absolu et séché.

On récupère 0,8g de 2-(perfluorohexyl)éthyl **β**-D-glucofuranuronate de sodium, soit un rendement calculé sur la base de la lactone de départ égal à 74 %.

### EXEMPLE 3

### Préparation de 2-(perfluorohexyl)éthyl β-D-glucofuranuronate de potassium.

On procède dans les conditions de l'exemple 2 modifié en ce que l'on remplace la lessive de soude par une solution d'hydroxyde de potassium 3N.

Le 2-(perfluorohexyl)éthyl **β**-D-glucofuranuronate de potassium obtenu présente une tension superficielle et une CMC respectivement égale à 22 mN/m (témoin = 16 mN/m) et 10 g/l.

### EXEMPLE 4

### Préparation de 2-(perfluorohexyl)éthyl D-glucofuranuronolactone-6(3).

Dans un ballon tricol de 500 ml, on introduit 12 g (68 mmoles) de D-glucuronolactone-6(3) (85, 145-0 ; ALDRICH), 39 g (84 mmoles) de 2-(perfluorooctyl)éthanol (FORALKYL EOH8 ; CECA S.A.), 50 ml de diglyme et 1,2 g (12,5 mmoles) d'acide méthanesulfonique.

Après 6 heures à 110°C sous un vide de 13,33 KPa, le milieu réactionnel est refroidi à 20°C, neutralisé par NaHCO₃ et agité en présence de 200 ml d'eau distillée.

Le précipité ainsi formé est filtré, lavé à l'eau (50 ml x 2) et à l'hexane chaud (60 ml x 3).

Le solide marron récupéré (28 g soit un rendement brut de 66 %) est solubilisé dans 7 ml d'acétate d'éthyle puis chromatographié sur une colonne de silice (230-400 Mesh ASTM; éluant: gradient acétate d'éthyle/hexane 70/30 (v/v) - acétate d'éthyle).

Les fractions correspondant à chacun des anomères α et β sont rassemblées et concentrées sous vide. Le rapport en poids de l'anomère α à l'anomère **β** est égal à 4/15.

Les caractéristiques des anomères α et β de la 2-(perfluorooctyl)éthyl D-glucofuranuronolactone-6(3) sont présentées ci-après.

| RMN¹H | | |
|---|---|---|
| | **Anomère α** | **Anomère β** |
| H1 | 5,32 ; d | 5,12 ; s |
| | J_{H1H2} = 4,49 | J_{H1H2} < 1,5 |
| | | |
| H4 | 4,85 ; m | 5,01 ; dxd |
| | | J_{H4H5} = 6,52 |
| | | J_{H4H3} = 4,64 |
| | | |
| H3 | 4,77 ; m | 4,86 ; d |
| | | J_{H3H4} = 4,64 |
| | | J_{H3H2} < 1 |
| | | |
| H5 | 4,65 ; m | 4,57 ; d |
| | | J_{H5H4} = 6,52 |
| | | |
| H2 | 4,27 ; m | 4,26 ; s |
| | | J_{H2H1} < 1,5 |
| | | _{H2H3} < 1 |
| -CH₂-O- | 4,15-4,00 et 4,00-3,85 | 4,23-4,10 et 3,77-3,65 |
| | | |
| -CH₂-CH₂-O- | 2,80-2,30 | 2,67-2,30 |

| RMN¹³C | | |
|---|---|---|
| | **Anomère α** | **Anomère β** |
| C = O | 175,29 | 175,70 |
| C1 | 104,17 | 109,80 |
| C3 | 85,44 | 84,10 |
| C2 | 77,43 | 79,42 |
| C4 | 77,99 | 79,00 |
| C5 | 70,62 | 70,23 |
| -CH₂-O- | 61,57 et 61,48 | 60,23 |
| -CF₂-CH₂- | 32,26-31,43 | 32,25-31,40 |

| IR | | |
|---|---|---|
| | **Anomère α** | **Anomère β** |
| δCH₂ | 2981-2872 | 2944 |
| γC = O | 1800-1741 | 1805, 1778 et 1757 |
| γ-OH | 3408 | |

### EXEMPLE 5

### Préparation de 2-(perfluorooctyl)éthyl β-D-glucofuranuronate de sodium.

A 6g (9,6 mmoles) de 2-(perfluorooctyl)éthyl **β**-D-glucofuranuronolactone-6(3) de l'exemple 4 solubilisée dans 150 ml d'éthanol à 96 %, on ajoute sous agitation et à 25°C une solution d'hydroxyde de sodium 2,86 N jusqu'à obtention d'un pH égal à 12.

Après 3 heures d'agitation à 25°C, et concentration partielle de la suspension sous vide, le précipité formé est filtré, lavé deux fois avec 20 ml d'éthanol absolu.

On récupère 4 g de 2-(perfluorooctyl)éthyl **β**-D-glucofuranuronate de sodium, soit un rendement calculé sur la base de la lactone de départ égal à 62 %.

### EXEMPLE 6

### Préparation de 2-(perfluorooctyl)éthyl β-D-glucofuranuronate de potassium.

On procède dans les conditions de l'exemple 5 modifié en ce que l'on remplace l'hydroxyde de sodium par l'hydroxyde de potassium 3N.

Le 2-(perfluorooctyl)éthyl **β**-D-glucofuranuronate de potassium obtenu présente une tension superficielle et une CMC respectivement égale à 21 mN/m (témoin = 16 mN/m) et 1,2 g/l.

### EXEMPLE 7

### Préparation de 11-(perfluorooctyl)undécyl D-glucofuranuronolactone-6(3).

Dans un ballon tricol de 250 ml, on introduit 8 g (45 mmoles) de D-glucuronolactone-6(3) (85, 145-0 ; ALDRICH), 32,8 g (55 mmoles) de 11-(perfluorooctyl)undécanol (CECA S.A.), 40 ml de diglyme et 0,9 g (9,35 mmoles) d'acide méthanesulfonique.

Après 8 heures à 110°C sous un vide de 13,33 KPa, on refroidit le milieu réactionnel à 25°C, on ajoute 200 ml d'eau permutée et on agite énergiquement pendant une heure.

Le précipité ainsi formé est filtré, lavé à l'eau permutée (100 ml x 2) et à l'éther éthylique (100 ml x 3).

Le solide récupéré (20 g soit un rendement de 58,8 %) est un mélange des anomères α et β de la 11-(perfluorooctyl)undécyl D-glucofuranuronolactone-6(3). Après séparation par chromatographie sur colonne (silice 230-400 Mesh ASTM; éluant: acétate d'éthyle/hexane 70/30 (v/v)), on récupère :
- 8 g de l'anomère β (rendement 23,5 %)
- 0,7 g de l'anomère α (rendement 0,9 %)
- et 5,1 g d'un mélange des anomères α et β (rendement 15 %)

Les caractéristiques des anomères α et β sont présentées ci-après.

| RMN¹H | | |
|---|---|---|
| | **Anomère α** | **Anomère β** |
| H1 | 5,21 ; d | 5,03 |
| | J_{H1H2} = 4,49 | J_{H1H2} < 1,5 |
| | | |
| H4 | 4,90-4,70 ; m | 4,95 |
| | | J_{H4H5} = 6,0 |
| | | |
| H3 | 4,90-4,70 ; m | 4,80 |
| | | J_{H3H4} = 4,6 |
| | | J_{H3H2} < 1 |
| | | |
| H5 | 4,60 ; m | 4,51 ; d |
| | | |
| H2 | 4,20 ; m | 4,23 |
| | | J_{H2H1} < 1,5 |
| | | J_{H2H3} < 1 |
| -CH₂-O- | 3,90-3,70 | 3,84-3,70 et 3,36-3,24 |
| | | |
| -CH₂-CH₂-O- | 3,45-3,65 | 2,40-2,10 |
| | | |
| Chaîne alkyle | 1,80-1,40 et 1,30 | 1,70-1,40 et 1,30 |

| RMN¹³C | | |
|---|---|---|
| | **Anomère α** | **Anomère β** |
| C = O | 175,00 | 175,21 |
| C1 | 103,91 | 110,25 |
| C3 | 85,52 | 83,84 |
| C2 | 77,09 | 78,99 |
| C4 | 77,74 | 78,73 |
| C5 | 79,69 | 70,20 |
| -CH₂-O- | 69,69 | 68,68 |
| -(CH₂)ₙ- | 33,84 ; 31,31 ; 30,87 ; 30,45 ; 30,33 ; 30,22 ; 30,03 ; 29,72 ; 26,75 ; 20,93 | 31,74 ; 31,31 ; 30,86 ; 30,29 ; 30,24 ; 30,20 ; 30,02 ; 29,71 ; 26,79 ; 20,92 |

| IR | | |
|---|---|---|
| | **Anomère α** | **Anomère β** |
| δCH₂ | 2960 | 2926,2855 |
| γC = O | 1770 | 1785 |

### EXEMPLE 8

### Préparation de 11-(perfluorooctyl)undécyl β-D-glucofuranuronate de sodium.

A 8 g (10,7 mmoles) de 11-(perfluorooctyl)undécyl **β**-D-glucofuranuronolactone-6(3) de l'exemple 7 solubilisée dans 150 ml d'éthanol à 96 % et maintenue à 35°C, on ajoute, goutte à goutte, une solution de soude 2,86 N jusqu'à l'obtention d'un pH voisin de 12.

Après 3 heures d'agitation, on concentre partiellement la suspension (jusqu'à 50 ml). Le précipité formé est filtré avec de l'éthanol absolu (20 ml x 2) et séché sous vide.

On récupère 4,15 g de 11-(perfluorooctyl)undécyl β-D-glucofuranuronate de sodium, soit un rendement calculé sur la base de la lactone de départ égal à 53 %.

Ce composé présente les caractéristiques en IR suivantes :
δCH₂ : 2922, 2853 et γC=O : 1625.

### EXEMPLE 9

### Préparation de 11-(perfluorooctyl)undécényl D-glucofuranuronolactone-6(3).

Dans un ballon tricol de 250 ml, on introduit 7,4 g (42 mmoles) de D-glucuronolactone-6(3) (85, 145-0 ; ALDRICH), 30 g (51 mmoles) de 11-(perfluorooctyl)undécénol (CECA S.A.), 70 ml (56,2 g) de diglyme et 0,8 g (54 µl ; 8,3 mmoles) d'acide méthanesulfonique.

Après 12 heures à 80°C sous un vide de 13,33 KPa (distillation de 10 ml de diglyme et d'eau), le milieu réactionnel est refroidi à 25°C. Après un ajout de 90 ml d'eau, on neutralise avec NaHCO₃ et on agite énergiquement pendant 10 minutes.

Le précipité pâteux ainsi obtenu est filtré, essoré et solubilisé dans 180 ml d'éther diéthylique.

Après lavage de la phase organique à l'eau (30 ml x 2), séchage avec du sulfate de magnésium et concentration sous vide, on obtient un résidu contenant un mélanges des anomères α et β de la 11-(perfluorooctyl)undécényl D-glucofuranuronolactone. Après séparation par chromatographie sur colonne (silice 230-400 Mesh ASTM ; éluant: gradient éther diéthylique/hexane 1/1 (v/v) - éther diéthylique), on récupère 19 g de l'anomère β et 2 g de l'anomère α, soit un rendement calculé sur la base de la lactone de départ égal à 60,6 % et 6,4 % respectivement.

Les caractéristiques des anomères α et β sont présentées ci-après.
- point de fusion de l'anomère α : 68-70°C
- Rf :
anomère *α :* 0,53 (forme Z) et 0,46 (forme E)
anomère β : 0,31 (forme Z) et 0,24 (forme E)

| RMN¹H | | |
|---|---|---|
| | **Anomère α** | **Anomère β** |
| CH=CH(1H) | 6,50-6,35 ; m 70 % | 6,50-6,35 ; m 72 % |
| | 6,20-6,05 ; m 30 % | 6,20-6,05 ; m 28 % |
| | | |
| CH=CH(1H) | 5,70-5,52 ; m 70 % | 5,70-5,52 ; m 72 % |
| | 5,52-5,40 ; m 30 % | 5,52-5,40 ; m 28 % |
| | | |
| H1 | 5,28 | 5,10 |
| | J_{H1H2} = 4,4 | J_{H1H2} < 1 |
| | | |
| H4 | 4,84; m | 5,01; m |
| | J_{H3H4} = 3,2 | J_{H4H5} = 5,8 |
| | J_{H4H5} = 4,8 | J_{H4H3} = 4,9 |
| | | |
| H3 | 4,78; m | 4,87 ; d |
| | J_{H3H2} < 1 | J_{H3H2} < 1 |
| | | |
| H5 | 4,49 ; m | 4,35 ; m |
| | J_{H5OH} = 8 | J_{H5OH} = 8,4 |
| | | |
| H2 | 4,40 ; t | 4,45 ; m |
| | J_{H2OH} = 5,3 | |
| | | |
| H1'a | 3,89 ; m | 3,68 ; m ; chaîne alkyle |
| | | |
| H1'b | 3,59 ; m | 3,42 ; m ; chaîne alkyle |
| | | |
| OH-2 | 3,08 ; d | |
| | J_{H2OH} = 5,4 | |
| | | |
| OH-5 | 2,68 ; d | 2,68 ; d |
| | J_{H5OH} = 8,5 | (J_{H5OH} = 5,3) |
| | | |
| H9'a(1H) | 2,35-2,25 ; m 30 % | 2,40-2,10 ; m |
| | 2,25-2,10 ; m 70 % | |
| | | |
| H2'a, 2'b | (2H) 1,65-1,58 ; m | |
| | | (15H) 1,65-1,20 ; m |
| H9'b, CH₂3', 8' | (13H) 1,45-1,30 | |

| RMN¹³C | | |
|---|---|---|
| | **Anomère α** | **Anomère β** |
| C6 | 174,45 | 174,85 |
| CH=CH | 145,65-144,54-143,37-117,33-116,87-116,42 | 145,66-143,38-117,25-116,79-116,34-116,04 |
| C1 | 102,59 | 109,20 |
| C3 | 84,81 | 83,34 |
| C4 | 76,39 | 77,13 |
| C2 | 76,03 | 77,57 |
| C5 | 70,37 | 69,33 |
| C1' | 69,84 | 69,18 |
| C2', 9' | 32,07-26,03 | 32,07-25,94 |

| IR | | |
|---|---|---|
| | **Anomère α** | **Anomère β** |
| δCH₂ | 2961 | 2927,2856 |
| γC = O | 1771 | 1791 |

### EXEMPLE 10

### Préparation de 11-(perfluorooctyl)undécényl β-D-glucofuranuronate de sodium.

A 0,241 g (0,33 mmoles) de 11-(perfluorooctyl)undécyl β-D-glucofuranuronolactone-6(3) de l'exemple 9 solubilisée dans 2 ml d'éthanol absolu, on ajoute 0,11 ml d'une solution de soude 2,86 N.

Après 2 heures d'agitation à 25°C, le précipité formé est filtré, lavé à l'éthanol absolu froid (5 ml x 2) et séché sous vide (2,4 KPa ; 40°C).

On récupère 160 mg de 11-(perfluorooctyl)undécényl **β**-D-glucofuranuronate de sodium (mélange des formes Z et E), soit un rendement calculé sur la base de la lactone de départ égal à 63 %.

Ce composé présente les caractéristiques en lR suivantes :
δCH₂ : 2923, 2856 ; γC=O : 1626 ; γ-OH : 3411

La tension superficielle et la CMC sont respectivement égales à 17,4 mN/m (témoin = 16 mN/m) et 0,01 g/l.

### EXEMPLE 11

### Préparation de 11-(perfluorooctyl)undécényl β-D-glucofuranuronate de potassium.

On procède dans les conditions de l'exemple 10 modifié en ce que l'on remplace la solution de soude par une solution d'hydroxyde de potassium 2N.

Le 11-(perfluorooctyl)undécényl **β**-D-glucofuranuronate de potassium obtenu présente une tension superficielle et une CMC respectivement égale à 17,3 mN/m (témoin = 16 mN/m) et 0,01 g/l.

### EXEMPLE 12

### Préparation de 2-(perfluorohexyl)éthyl D-galacturonate de 2-(perfluorohexyl)éthyle.

Dans un ballon tricol de 500 ml, on introduit 8,48 g (0,04 mole) d'acide D-galacturonique monohydraté (85, 728-9; ALDRICH), et 87,4 g (0,24 mole) de 2-(perfluorohexyl)éthanol (FORALKYL EOH8 ; CECA S.A.).

On porte le mélange à 80°C sous un vide de 8 KPa et, après 15 minutes d'agitation, on ajoute 0,38 g (2 x 10⁻³ mole) d'acide paratoluènesulfonique monohydraté.

Après 12 heures, le milieu réactionnel est refroidi à 20°C, neutralisé par NaHCO₃ et filtré. Le filtrat, après concentration sous vide, fournit une huile brune contenant le 2-(perfluorohexyl)éthyl D-galacturonate de 2-(perfluorohexyl)éthyle sous la forme d'un mélange de l'anomère β-furanose (majoritaire), de l'anomère α-pyranose et de traces des anomères α-furanose et β-pyranose.

Le Rf des composés du mélange est le suivant :
- forme furanose : anomère α = 0,54
   anomère β = 0,63
- forme pyranose : anomère α = 0,26
   anomère β = 0,33

Cette huile est fluidifiée par l'ajout de 3 ml d'acétate d'éthyle (volume final : 35 ml) et chromatographiée sur colonne (silice 230-400 Mesh ASTM; éluant: acétate d'éthyle/hexane 70/30 (v/v)). On récupère 9 g de la forme β-furanose, soit un rendement calculé sur la base de l'acide D-galacturonique de départ égal à 25,4 %.

### EXEMPLE 13

### Préparation de 2-(perfluorooctyl)éthyl D-galacturonate de 2-(perfluorooctyl)éthyle.

Dans un ballon tricol de 250 ml, on introduit 4,8 g (0,023 mole) d'acide D-galacturonique monohydraté (85, 728-9; ALDRICH), 19,1 g (0,042 mole) de 2-(perfluorooctyl)éthanol (CECA S.A.), 40 ml de diglyme et 0,5 g (5x10⁻³ mole) d'acide méthanesulfonique.

Après 9 heures à 100°C sous un vide de 8 KPa, le milieu réactionnel est refroidi, neutralisé par NaHCO₃ et filtré. Le filtrat auquel on ajoute 100 ml d'eau permutée est agité énergiquement. Le précipité obtenu est filtré, lavé (150 ml d'eau permutée et 150 ml d'hexane chaud) et séché sous vide.

On récupère 17 g d'une poudre contenant le 2-(perfluorooctyl)éthyl D-galacturonate de 2-(perfluorooctyl)éthyle qui est un mélange des 4 anomères α et β des formes furanose et pyranose, la forme β-furanose étant majoritaire.

Ce mélange présente les caractéristiques en IR suivantes :
δCH₂ : 2920 ; γC=O : 1749 ; γ-OH : 3486

### EXEMPLE 14

### Préparation de 11-(perfluorooctyl)undécyl D-galacturonate de 11-(perfluorooctyl)undécyle.

Dans un ballon tricol de 250 ml, on introduit 2,9 g (0,014 mole) d'acide D-galacturonique monohydraté (85, 728-9; ALDRICH), 21,2g (0,036 mole) de 11-(perfluorooctyl)undécanol (CECA S.A.), 40 ml de diglyme et 0,3 g (3 x 10⁻³ mole) d'acide méthanesulfonique.

Après 9 heures à 100°C sous un vide de 9,33 KPa, le milieu réactionnel est refroidi et neutralisé avec NaHCO₃. On ajoute 120 ml d'eau permutée et on agite énergiquement pendant 30 minutes. Le précipité obtenu est filtré, lavé à l'eau permutée (100 ml x 2) et à l'hexane chaud (100 ml x 2) et séché sous vide.

On récupère 17 g d'un précipité contenant le 11-(perfluorooctyl)undécyl D-galacturonate de 11-(perfluorooctyl)undécyle, soit un rendement calculé sur la base de l'acide D-galacturonique de départ égal à 75 %.

Le précipité est un mélange des 4 anomères α et β des formes furanose et pyranose dont les caractéristiques en lR suivantes :
δCH₂ : 2923, 2853 ; γC=O : 1747 ; γ-OH : 3442

### EXEMPLE 15

### Préparation de 11-(perfluorooctyl)undécényl D-galacturonate de 11-(perfluorooctyl)undécényle.

Dans un ballon tricol de 250 ml, on introduit 3,6 g (0,017 mole) d'acide D-galacturonique monohydraté (85, 728-9; ALDRICH), 26,2 g (0,045 mole) de 11-(perfluorooctyl)undécénol (CECA S.A.), 40 ml de diglyme et 0,4 g (4 x 10⁻³ mole) d'acide méthanesulfonique.

Après 8 heures à 90°C sous un vide de 9,33 KPa, le milieu réactionnel est neutralisé avec NaHCO₃. On ajoute 150 ml d'eau permutée et on agite énergiquement pendant 30 minutes. Le précipité obtenu est filtré, lavé à l'eau permutée (100 ml x 3) et à l'hexane chaud (100 ml x 3).

On récupère 17g de 11-(perfluorooctyl)undécényl D-galacturonate de 11-(perfluorooctyl)undécényle, soit un rendement calculé sur la base de l'acide D-galacturonique de départ égal à 75 %.

Ce composé est solubilisé dans 5 ml d'acétate d'éthyle et filtré sur une colonne de silice (230-400 Mesh ASTM ; éluant : acétate d'éthyle). On récupère 10 g d'une huile orangée très visqueuse, correspondant au mélange des 4 isomères *a* et β des formes furanose et pyranose (rendement : 44 %).

Les caractéristiques en IR du mélange obtenu sont les suivantes :
δCH₂ : 2932, 2858 ; γC=O : 1740 ; γ-OH : 3391

### EXEMPLE 16

On prépare un shampooing en mélangeant les composés suivants (en g) :

| | |
|---|---|
| Composé selon l'exemple 2 | 5 |
| Composé selon l'exemple 12 | 1 |
| Lauryl éther sulfate de sodium (REWOPOL NL 3,28 ; SCHERING S.A.) | 40 |
| Cocoamidopropylbétaine (REWOTERIC AMB 13 ; SCHERING S.A.) | 2 |
| Alkyl polyglucoside 1200 (PLANTAREN 1200 ; HENKEL) | 2 |
| Chlorure de sodium | 1 |
| Conservateur (PHENONIP ; NIPA LABORATORIES) | 0,1 |
| Parfum (Fougère 10105, hydrosoluble 337 201 ; LASERSON et SABETAY) | 0,2 |
| Eau déminéralisée qsp 100 | |

### EXEMPLE 17

On prépare un après-shampooing en mélangeant les composés suivants (en g):

| | |
|---|---|
| Composé selon l'exemple 5 | 2 |
| MERQUAT 280* (Merck) | 5 |
| Conservateur (PHENONIP ; NIPA LABORATORIES) | 0,1 |
| Parfum (1/3 vanille 27800, hydrosoluble 008201 ; 2/3 fougère 10105, hydrosoluble 337201 ; LASERSON et SABETAY) | 0,1 |
| Eau déminéralisée qsp 100 | |

| | |
|---|---|
| * copolymère du chlorure de diméthyl dialkyl ammonium et de l'acide acrylique en solution aqueuse à 35 %. | |

## Revendications

1. Composés de formule : 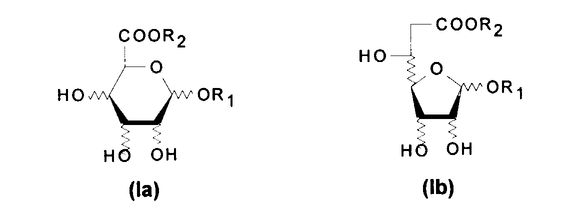 dans laquelle :
R₁ représente un radical fluoroalkyle, linéaire ou ramifié, saturé ou insaturé renfermant 4 à 46 atomes de carbone ;
R₂ représente H, R₁ tel que défini ci-avant, un métal alcalin ou alcalino-terreux, ou un ammonium quaternaire de formule : 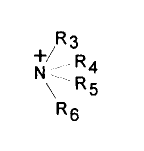 dans laquelle R₃, R₄, R₅ et R₆, identiques ou différents représentent H, un radical alkyle ou hydroxyalkyle en C₁C₆.

2. Composés selon la revendication 1, caractérisés en ce que R₁ a pour formule :
-(CH₂)ₘ-(CH=CH)ₙ-(CF₂)ₚ-CF₃
dans laquelle :
m est compris entre 2 et 22,
n est égal à 0 ou 1,
p est compris entre 1 et 21.

3. Composés de formule : 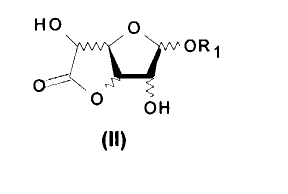 dans laquelle R₁ représente un radical fluoroalkyle linéaire ou ramifié, saturé ou insaturé, renfermant 4 à 46 atomes de carbone.

4. Composés selon la revendication 1, caractérisés en ce que R₁ a pour formule :
-(CH₂)ₘ-(CH=CH)ₙ-(CF₂)ₚ-CF₃
dans laquelle :
m est compris entre 2 et 22,
n est égal à 0 ou 1,
p est compris entre 1 et 21.

5. Composés selon l'une des revendications 3 ou 4, caractérisés en ce qu'il s'agit de la fluoroalkyl glucofuranuronolactone-6(3), de la fluoroalkyl mannofuranuronolactone-6(3), de la fluoroalkyl idofuranuronolactone-6(3) ou de la fluoroalkyl gulofuranuronolactone-6(3).

6. Procédé de préparation des composés selon l'une des revendications 1 à 3, caractérisé en ce qu'il comporte les étapes suivantes :
**a**- réaction d'un acide glycosiduronique ou d'une glycosiduronolactone avec un alcool de formule R₁OH dans laquelle R₁ représente un radical fluoroalkyle, linéaire ou ramifié, saturé ou insaturé, renfermant de 4 à 46 atomes de carbone pour former le fluoroalkylglycosiduronate de fluoroalkyle ou la fluoroalkylglycosiduronolactone;
**b**- le cas échéant, basification pour former le sel de l'acide fluoroalkylglycosiduronique, et
**c**- le cas échéant, acidification pour former l'acide fluoroalkylglycosiduronique.

7. Procédé selon la revendication 6, caractérisé en ce que l'acide glucosiduronique est choisi parmi les acides uroniques contenant 5 ou 6 atomes de carbone.

8. Procédé selon la revendication 7, caractérisé en ce que l'acide est l'acide glucuronique, galacturonique, mannuronique, iduronique ou guluronique.

9. Procédé selon la revendication 6, caractérisé en ce que la glycosiduronolactone est choisi parmi les lactones des acides uroniques renfermant 5 ou 6 atomes de carbone.

10. Procédé selon la revendication 9, caractérisé en ce que la glycosiduronolactone est la glucuronolactone-6(3), la mannuronolactone-6(3), la guluronolactone-6(3), et l'iduronolactone-6(3).

11. Utilisation des composés selon l'une des revendications 1 ou 3 comme agent tensio-actif, par exemple pour le lavage des vitres et des sols, et pour le nettoyage des composants électroniques.

12. Utilisation des composés selon l'une des revendications 1 ou 3 pour préparer des shampooings et après-shampooings, des compositions capillaires, des pommades, des crèmes et laits de beauté, des bains moussants et des compositions pour l'hygiène buccale.

13. Utilisation des composés selon l'une des revendications 1 ou 3 pour le traitement anti-buée.

14. Utilisation des composés selon l'une des revendications 1 ou 3 pour le traitement antistatique.

15. Utilisation des composés selon l'une des revendications 1 ou 3 pour le polissage de surface.

16. Utilisation des composés selon l'une des revendications 1 ou 3 pour préparer des poudres et mousses anti-feu.

17. Utilisation des composés selon l'une des revendications 1 ou 3 pour préparer des adhésifs, des revêtements de surface et des cires ou graisses de lubrification.

18. Utilisation des composés selon l'une des revendications 1 ou 3 en tant qu'additif de bains chromiques, d'imperméabilisation, de substitut du mercure dans les batteries à anode de zinc ou agent de polymérisation.

19. Utilisation des composés selon l'une des revendications 1 ou 3 en tant que cristaux liquides.

20. Utilisation des composés selon l'une des revendications 1 ou 3 dans le domaine biomédical, notamment pour stabiliser et vectoriser des principes actifs, et pour transporter l'oxygène.
